# EUROPEAN PATENT APPLICATION

(11) **EP 4 559 574 A1**
(43) Date of publication of application: **28.05.2025**
(21) Application number: 24155773.5
(22) Date of filing: 05.02.2024
(51) Int. Cl.: B01J 23/78, B01J 35/00, B01J 37/00, B01J 37/03

(54) **IRON-BASED CATALYST AND METHOD OF HYDROGENATING CARBON DIOXIDE**

(30) Priority: 22.11.2023 TW 112145076
(71) Applicant: Industrial Technology Research Institute, Chutung, Hsinchu 310401 (TW)
(72) Inventor: WU, Kuo-Ching, Kinmen County (TW); LIN, Pao-Tsern, Hsinchu County (TW); HSU, Hsi-Yen, Hsinchu City (TW); CHEN, Chao-Huang, Hsinchu City (TW)
(74) Representative: Michalski Hüttermann & Partner Patentanwälte mbB

(57) **Abstract**

A method of hydrogenating carbon dioxide, including contacting carbon dioxide and hydrogen with an iron-based catalyst to form a liquid and a gas. The liquid includes CₙH₂ₙ, CₙH₂ₙ₊₂, or a combination thereof and water, wherein n is 5 to 18. The gas includes CH₄, CₘH₂ₘ, CₘH₂ₘ₊₂, or a combination thereof, hydrogen, and carbon dioxide, wherein m is 2 to 9. The iron-based catalyst includes 70 mol% to 97 mol% of porous FeO(OH)ₓ (wherein 1<x<2), and 3 mol% to 30 mol% of alkaline metal compound loaded onto the porous FeO(OH)ₓ.

## Description

### TECHNICAL FIELD

The technical field relates to an iron-based catalyst for directly converting carbon dioxide to alkanes/olefins.

### BACKGROUND

Carbon monoxide serving as a major raw material in a conventional hydrogenation process can be reacted with hydrogen to form what is called syngas, or synthesis gas. This CO/H₂ gas mixture is reacted to form petrochemical alkanes/olefins such as gasoline and diesel. There is no commercial process at present for directly converting carbon dioxide to alkanes/olefins. When carbon dioxide is converted to alkanes/olefins, the carbon dioxide should firstly be converted to carbon monoxide, and the carbon monoxide is then reacted with hydrogen to form alkanes/olefins.

Accordingly, a novel catalyst and a corresponding conversion condition is called for directly converting carbon dioxide and hydrogen to alkanes/olefins, but not convert to carbon monoxide and then to alkanes/olefins.

### SUMMARY

One embodiment of the disclosure provides an iron-based catalyst, including: 70 mol% to 97 mol% of porous FeO(OH)ₓ, wherein 1<x<2; and 3 mol% to 30 mol% of alkaline metal compound loaded on the porous FeO(OH)ₓ.

One embodiment of the disclosure provides a method of hydrogenating carbon dioxide, including: (i) contacting carbon dioxide and hydrogen with an iron-based catalyst to form a liquid and a gas, wherein the liquid includes CₙH₂ₙ, CₙH₂ₙ₊₂, or a combination thereof and water, and n is 5 to 18, wherein the gas includes CH₄, CₘH₂ₘ, CₘH₂ₘ₊₂, or a combination thereof, hydrogen, and carbon dioxide, and m is 2 to 9, wherein the iron-based catalyst includes 70 mol% to 97 mol% of porous FeO(OH)ₓ, wherein 1<x<2; and 3 mol% to 30 mol% of alkaline metal compound loaded on the porous FeO(OH)ₓ.

In some embodiments, the method further includes: (ii) separating the gas and the liquid; and (iii) contacting the gas with another iron-based catalyst to form another liquid and another gas, wherein the other liquid includes CₙH₂ₙ, CₙH₂ₙ₊₂, or a combination thereof and water, and n is 5 to 18, wherein the other gas includes CH₄, CₘH₂ₘ, CₘH₂ₘ₊₂, or a combination thereof, hydrogen, and carbon dioxide, and m is 2 to 9, wherein the other iron-based catalyst includes: 70 mol% to 97 mol% of another porous FeO(OH)ₓ, wherein 1<x<2; and 3 mol% to 30 mol% of another alkaline metal compound loaded on the other porous FeO(OH)ₓ.

A detailed description is given in the following embodiments with reference to the accompanying drawings.

### BRIEF DESCRIPTION OF the DRAWINGS

The disclosure can be better understood by reading the subsequent detailed descriptions and examples with references made to the accompanying drawings, wherein:
Fig. 1 shows a Raman spectrum of iron-based support in one embodiment of the disclosure.
Fig. 2 shows a Raman spectrum of iron-based support in one embodiment of the disclosure.
Fig. 3 shows a Raman spectrum of iron-based support in one embodiment of the disclosure.

### DETAILED DESCRIPTION

In the following detailed descriptions, for the purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

One embodiment of the disclosure provides an iron-based catalyst, including 70 mol% to 97 mol% of porous FeO(OH)ₓ, wherein 1<x<2; and 3 mol% to 30 mol% of alkaline metal compound loaded on the porous FeO(OH)ₓ. If x=0, the iron-based support will be FeO (i.e. iron oxide), and the iron-based catalyst cannot directly hydrogenate carbon dioxide to alkanes/olefins. If the amount of alkaline metal compound is too high, the conversion rate of the carbon dioxide will be too low to be efficiently converted to the alkanes/olefins. If the amount of alkaline metal compound is too low, the amount of the intermediate products such as carbon monoxide will be too high. In general, the iron-based support is substantially free of iron oxides such as FeO, Fe₂O₃, Fe₃O₄, or the like, which can be determined by the Raman spectrum of the iron-based support. In general, the iron oxide will deteriorate the capability of the iron-based support to directly convert the carbon dioxide to alkanes/olefins.

In some embodiments, the porous FeO(OH)ₓ has a specific surface area of at least 100 m²/g, usually in the range of 100 m²/g to 300 m²/g. If the specific surface area of the porous FeO(OH)ₓ is too small, the available reactive surface area will be too small, resulting in insufficient catalyst performance. With current synthesis technology, it is not easy to synthesize porous FeO(OH)ₓ having a surface area higher than 300 m²/g. In some embodiments, the porous FeO(OH)ₓ has a pore volume of 0.2 cm³/g to 0.5 cm³/g. If the pore volume of the porous FeO(OH)ₓ is too small, the surface area and activity of the porous FeO(OH)ₓ will decline. In some embodiments, the porous FeO(OH)ₓ has an average pore size of 40 Å to 70 Å. If the average pore size of porous FeO(OH)ₓ is too large, the surface area and activity of the porous FeO(OH)ₓ will decline.

In some embodiments, the alkaline metal compound includes sodium carbonate, potassium carbonate, sodium hydroxide, potassium hydroxide, sodium oxide, potassium oxide, or a combination thereof. In general, the alkaline metal compound is common alkaline compound such as sodium carbonate, potassium carbonate, sodium hydroxide, potassium hydroxide, or a combination thereof. However, a part of the carbonate may decompose to carbon dioxide and oxides during the heating process, and a part of the hydroxide may dehydrate into oxides during the heating process.

In some embodiments, the iron-based catalyst can be formed by the following steps. First, an alkaline solution (such as ammonia, sodium hydroxide aqueous solution, potassium hydroxide aqueous solution, sodium carbonate aqueous solution, or potassium carbonate aqueous solution) is rapidly mixed with an iron salt aqueous solution (such as iron nitrate or iron hydroxide aqueous solution). For example, the alkaline solution and the iron salt aqueous solution can be stirred at medium to high speed (e.g. >1000 rpm) for a short period of time (e.g. less than 5 minutes) to be rapidly mixed. Alternatively, the rapid mixing can be performed by any other method and is not limited to stirring at high speed. If the mixing speed is not fast enough, the iron-based support will have a specific surface area that is too low, a pore volume that is too small, and an average pore size that is too large.

Subsequently, the pH value of the mixture is adjusted to 8 to 10 (e.g. 9) and rested for 1 to 3 hours in order to precipitate. If the pH value is too low, the particle size and the average pore size of the precipitate will be too large. If the pH value of the mixture is too high, the precipitate cannot be formed efficiently. The precipitate is then filtered out, and the filtered cake is washed out with water and baked at 100°C to 110°C to obtain an iron-based support, i.e. FeO(OH)ₓ. If the alkaline solution is ammonia, the iron-based support should be further immersed into an aqueous solution of an alkaline metal compound such as sodium hydroxide, potassium hydroxide, sodium carbonate, or potassium carbonate and then dried for loading the alkaline metal compound onto the iron-based support. If the alkaline solution is sodium hydroxide aqueous solution, potassium hydroxide aqueous solution, sodium carbonate aqueous solution, or potassium carbonate aqueous solution, the loading step can be optionally omitted. Finally, the iron-based support having the alkaline metal compound loaded thereon is dried and shaped to obtain the iron-based catalyst. If the baking and drying temperature is too high, Fe₂O₃ or Fe₃O₄ will be produced to deteriorate the performance of the iron-based catalyst. If the baking and drying temperature is too low, FeO(OH)ₓ will not be efficiently produced.

In general, the iron salt reacts with the alkaline to form iron hydroxide (Fe(OH)₃). After heating, an intramolecular dehydration reaction will occur for the iron hydroxide to form Fe₂O(OH)₄, which may further be dehydrated to form FeO(OH)ₓ. FeO(OH)ₓ can be further dehydrated to form Fe₂O₃ or Fe₃O₄. It can be deduced from the TGA analysis spectrum that when the iron hydroxide (Fe(OH)₃) is heated to 50°C, intramolecular dehydration occurs, which induces Fe₂O(OH)₄ formation. When Fe₂O(OH)₄ is further heated to about 100°C to 110°C, FeO(OH)ₓ is formed. As shown by the Raman spectra, there are almost no signals of Fe₂O₃ and Fe₃O₄ in FeO(OH)ₓ. If FeO(OH)ₓ is further heated to a higher temperature (e.g. higher than 200°C), Fe₂O₃ or Fe₃O₄ will be formed. The mole number of Fe and the number of x of the formed FeO(OH)ₓ can be calculated from the initial weight of the iron salt. For example, if the mole number of Fe is 1 mole and the whole product is FeO(OH), the product weight should be 88.85 g. If the mole number of Fe is 1 mole and the whole product is FeO(OH)₂, the product weight should be 105.86 g. As such, when the product in practice has a weight of 97.35 g, x number of the FeO(OH)ₓ will be about 1.5, i.e. FeO(OH) and FeO(OH)₂ have a molar ratio of about 1:1.

One embodiment of the disclosure provides a method of hydrogenating carbon dioxide, including: (i) contacting carbon dioxide and hydrogen with the described iron-based catalyst to form a liquid and a gas, wherein the liquid includes CₙH₂ₙ, CₙH₂ₙ₊₂, or a combination thereof and water, and n is 5 to 18, wherein the gas includes CH₄, CₘH₂ₘ, CₘH₂ₘ₊₂, or a combination thereof, hydrogen, and carbon dioxide, and m is 2 to 9. In some embodiments, the hydrogen and the carbon dioxide have a ratio of 2:1 to 4:1, and the hydrogen and the carbon dioxide have a gas hourly space velocity (GHSV) of 300 hr⁻¹ to 3000 hr⁻¹. If the amount of hydrogen is too high, the amount of un-reacted hydrogen will be too high, which wastes resources. If the amount of hydrogen is too low, the amount of hydrogen will be insufficient, resulting in an overly low conversion rate of the carbon dioxide. If the gas hourly space velocity of the hydrogen and the carbon dioxide is too high, the contact time will be insufficient to form the alkanes/olefins. If the gas hourly space velocity of the hydrogen and the carbon dioxide is too low, the catalyst efficiency will be low and the economic benefit will be insufficient.

In some embodiments, the step of contacting the carbon dioxide and the hydrogen with the iron-based catalyst is performed under a pressure of 50 to 400 psi, and at a temperature of 260 to 360°C. If the pressure is too high, the energy consumption will be too high. If the pressure is too low, the multi-carbon alkanes/olefins cannot be efficiently formed. If the temperature is too high, carbon monoxide will be easily formed. If the temperature is too low, the carbon dioxide cannot react efficiently.

In some embodiments, the conversion rate of the carbon dioxide is 50 to 80%, and the yield of CₙH₂ₙ, CₙH₂ₙ₊₂, or a combination thereof, and the yield of CH₄, CₘH₂ₘ, CₘH₂ₘ₊₂, or a combination thereof is 45 to 75%. Note that the conventional methods of directly converting carbon dioxide (not carbon monoxide) to alkanes/olefins (e.g. CₙH₂ₙ, CₙH₂ₙ₊₂, or a combination thereof and CH₄, CₘH₂ₘ, CₘH₂ₘ₊₂, or a combination thereof) usually have conversion rates far below 50%.

Alternatively, the method may further includes (ii) separating the gas and the liquid; and (iii) contacting the gas with another iron-based catalyst to form another liquid and another gas. The other liquid includes CₙH₂ₙ, CₙH₂ₙ₊₂, or a combination thereof and water, and n is 5 to 18. The other gas includes CH₄, CₘH₂ₘ, CₘH₂ₘ₊₂, or a combination thereof, hydrogen, and carbon dioxide, and m is 2 to 9. The other iron-based catalyst includes 70 mol% to 97 mol% of another porous FeO(OH)ₓ, wherein 1<x<2; and 3 mol% to 30 mol% of another alkaline metal compound loaded on the other porous FeO(OH)ₓ.

Compared to the method with a single step (i), the method performing steps (i) to (iii) may further increase the conversion rates of carbon dioxide and hydrogen. For example, the conversion rate of carbon dioxide of step (i) is at most 80%, but the conversion rate of carbon dioxide of steps (i) to (iii) may exceed 80% or even exceed 95%. The method with a single step (i) needs further treatment to separate the unreacted CO₂ and H₂ from the gaseous product CH₄, CₘH₂ₘ, CₘH₂ₘ₊₂, or a combination thereof, thereby increasing the separation cost. The method with steps (i) to (iii) may greatly decrease the unreacted CO₂ and H₂, thereby reducing the separation cost. In addition, the method with a single step (i) mainly produces the gaseous CH₄, CₘH₂ₘ, CₘH₂ₘ₊₂, or a combination thereof (m is mainly 2 to 3). The method performing steps (i) to (iii) may significantly increase the liquid product having a high carbon number such as CₙH₂ₙ, CₙH₂ₙ₊₂, or a combination thereof (n is 5 to 18) and gaseous product having a high carbon number such as CₘH₂ₘ, CₘH₂ₘ₊₂, or a combination thereof (m>4).

In some embodiments, step (ii) utilizes a gas-liquid separation tank to separate the gas and the liquid. In some embodiments, step (ii) may cool the gas and the liquid generated from step (i) to below 100°C (e.g. 5°C to 80°C), thereby separating the liquid (e.g. water and CₙH₂ₙ, CₙH₂ₙ₊₂, or a combination thereof) and the gas (e.g. unreacted CO₂ and H₂ and CH₄, CₘH₂ₘ, CₘH₂ₘ₊₂, or a combination thereof) generated from step (i). Subsequently, the liquid is collected, and the separated gas is contacted with the other iron-based catalyst in the next reactor, to form another liquid and another gas as in step (iii).

In some embodiments, the types and the amounts of the iron-based catalysts, the reaction temperatures, and the reaction pressures in steps (iii) and (i) are the same or similar. In some embodiments, the types and the amounts of the iron-based catalysts, the reaction temperatures, and the reaction pressures in steps (iii) and (i) are different. For example, the conversion rate of carbon dioxide in step (iii) is higher than or equal to the conversion rate of carbon dioxide in step (i), thereby further increasing the liquid product having a high carbon number such as CₙH₂ₙ, CₙH₂ₙ₊₂, or a combination thereof (n is 5 to 18) and gaseous product having a high carbon number such as CₘH₂ₘ, CₘH₂ₘ₊₂, or a combination thereof (m>4).

In some embodiments, the method may repeat steps (ii) and (iii) several times. For example, after separating the liquid and the gas, the gas may contact the iron-based catalyst in a next reactor to form new liquid and gas. Step (ii) separating the liquid and the gas and step (iii) contacting the separated gas with the iron-based catalyst in the next reactor can be repeated several times, until the produced gas is substantially free of carbon dioxide, hydrogen.

Accordingly, the method of fabricating the iron-based catalyst in the disclosure is simple and suits for mass production. This iron-based catalyst can be efficient to directly convert the carbon dioxide to the alkanes/olefins.

Below, exemplary embodiments will be described in details with reference to accompanying drawings so as to be easily realized by a person having ordinary knowledge in the art. The inventive concept may be embodied in various forms without being limited to the exemplary embodiments set forth herein. Descriptions of well-known parts are omitted for clarity.

### EXAMPLES

### Preparation Example 1

An aqueous solution of 2.2 mole of iron nitrate and ammonia were stirred at high speed to mix for 3 minutes, and the pH value of the mixture was then adjusted to 9. The mixture was then continuously stirred for 120 minutes and then filtered out. The filtered cake was washed out with water and then baked to obtain an iron-based support such as porous FeO(OH)ₓ. The iron-based support was baked at 110°C and then measured its weight (200 g), thereby calculating the FeO(OH) content (77 mol%) and the FeO(OH)₂ content (23 mol%), i.e. x in the product FeO(OH)x was 1.23.

### Preparation Example 2

An aqueous solution of 3.6 mole of iron nitrate and ammonia were stirred at high speed to mix for 3 minutes, and the pH value of the mixture was then adjusted to 9. The mixture was then continuously stirred for 120 minutes and then filtered out. The filtered cake was washed out with water and then baked to obtain an iron-based support such as porous FeO(OH)ₓ. The iron-based support was baked at 110°C and then measured its weight (332 g), thereby calculating the FeO(OH) content (63 mol%) and the FeO(OH)₂ content (37 mol%), i.e. x in the product FeO(OH)x was 1.37.

### Preparation Example 3

An aqueous solution of 18.5 mole of iron nitrate and ammonia were stirred at high speed to mix for 3 minutes, and the pH value of the mixture was then adjusted to 9. The mixture was then continuously stirred for 120 minutes and then filtered out. The filtered cake was washed out with water and then baked to obtain an iron-based support such as porous FeO(OH)ₓ. The iron-based support was baked at 110°C and then measured its weight (1730 g), thereby calculating the FeO(OH) content (48 mol%) and the FeO(OH)₂ content (52 mol%), i.e. x in the product FeO(OH)x was 1.52.

### Preparation Example 4

An aqueous solution of 60 mole of iron nitrate and ammonia were stirred at high speed to mix for 3 minutes, and the pH value of the mixture was then adjusted to 9. The mixture was then continuously stirred for 120 minutes and then filtered out. The filtered cake was washed out with water and then baked to obtain an iron-based support such as porous FeO(OH)ₓ. The iron-based support was baked at 110°C and then measured its weight (5620 g), thereby calculating the FeO(OH) content (46 mol%) and the FeO(OH)₂ content (54 mol%), i.e. x in the product FeO(OH)x was 1.54.

### Example 1

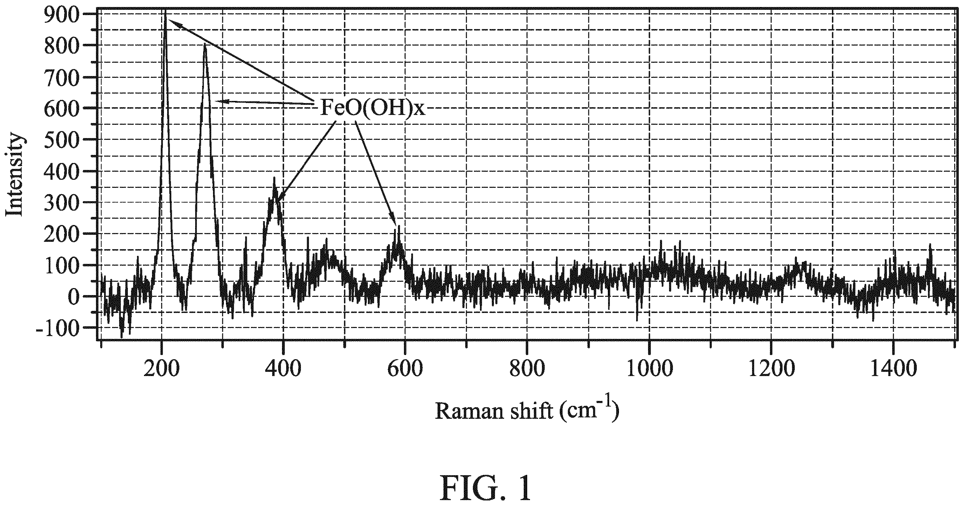

The iron-based support in Preparation Example 3 was selected, which had a Raman spectrum as shown in Fig. 1, which is almost free of the signals of Fe₂O₃ and Fe₃O₄. The obtained iron-based support such as FeO(OH)ₓ had a specific surface area of 249 m²/g (measured and calculated by BET method, such as nitrogen adsorption at constant temperature), a pore volume of 0.39 cm³/g (measured and calculated by BJH method, such as nitrogen adsorption at constant temperature), and an average pore size of 62 Å (measured and calculated by BJH method, such as nitrogen adsorption at constant temperature). The porous FeO(OH)ₓ was then added into a potassium carbonate aqueous solution, such that the potassium carbonate was loaded on the porous FeO(OH)ₓ. The above substance was then baked at 110°C and shaped to obtain an iron-based catalyst, wherein the molar percentage of the FeO(OH)ₓ was 95 mol% (x=1.52), and that of the potassium carbonate was 5 mol%.

The iron-based catalyst was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 350°C under a reaction pressure of 200 psi. The gas composition analysis (analyzed by Gas Chromatography, such as simultaneously measured and calculated through a column Carboxen-1010 PLOT, 30 m * 0.53 mm ID with TCD detector, and a column VB-1, 30 m * 0.53 mm ID with FID detector. The same analysis method will be applied in the following examples) of the reaction revealed that, the conversion rate of the carbon dioxide was 66.0%, the yield of carbon monoxide was 1.5%, the yield of methane was 32.1%, the yield of ethene (C₂H₄) was 11.4%, the yield of ethane (C₂H₆) was 3.6%, and the yield of propene/propane (C₃H₆/C₃H₈) was 17.5%. The yield of C₁₋₃ alkanes/olefins was 64.6%.

### Example 2-1

The iron-based support of porous FeO(OH)ₓ in Preparation Example 3 was added into a potassium carbonate aqueous solution, such that the potassium carbonate was loaded on the porous FeO(OH)ₓ. The above substance was then baked at 110°C and shaped to obtain an iron-based catalyst, wherein the molar percentage of the FeO(OH)ₓ was 75 mol% (x=1.52), and that of the potassium carbonate was 25 mol%.

The iron-based catalyst was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of 200 psi. The gas composition analysis of the reaction revealed that, the conversion rate of the carbon dioxide was 74.0%, the yield of carbon monoxide was 1.9%, the yield of methane was 35.4%, the yield of ethene was 14.1%, the yield of ethane was 4.5%, and the yield of propene/propane was 16.4%. The yield of C₁₋₃ alkanes/olefins was 70.4%.

### Example 2-2 (lower limit of H₂/CO)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=2/1, mol/mol) was introduced into the reactor at a reaction temperature of 320°C under a reaction pressure of 200 psi. The gas composition analysis of the reaction revealed that, the conversion rate of the carbon dioxide was 43.0%, the yield of carbon monoxide was 4.3%, the yield of methane was 19.8%, the yield of ethene was 6.8%, the yield of ethane was 2.6%, and the yield of propene/propane was 9.5%. The yield of C₁₋₃ alkanes/olefins was 38.7%.

### Example 2-3 (excessive H₂/CO)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=6/1, mol/mol) was introduced into the reactor at a reaction temperature of 320°C under a reaction pressure of 200 psi. The gas composition analysis of the reaction revealed that, the conversion rate of the carbon dioxide was 84.7%, the yield of carbon monoxide was 0.7%, the yield of methane was 51.2%, the yield of ethene was 6.1%, the yield of ethane was 9.2%, and the yield of propene/propane was 17.5%. The yield of C₁₋₃ alkanes/olefins was 84.0%.

### Example 2-4 (inadequate H₂/CO)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=1/1, mol/mol) was introduced into the reactor at a reaction temperature of 320°C under a reaction pressure of 200 psi. The gas composition analysis of the reaction revealed that, the conversion rate of the carbon dioxide was 15.3%, the yield of carbon monoxide was 6.7%, the yield of methane was 5.9%, the yield of ethene was 0.7%, the yield of ethane was 0.4%, and the yield of propene/propane was 1.6%. The yield of C₁₋₃ alkanes/olefins was 8.6%.

### Example 2-5 (upper limit of reaction temperature)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 360°C under a reaction pressure of 200 psi. The gas composition analysis of the reaction revealed that, the conversion rate of the carbon dioxide was 79.4%, the yield of carbon monoxide was 2.8%, the yield of methane was 37.3%, the yield of ethene was 13.7%, the yield of ethane was 5.5%, and the yield of propene/propane was 20.1%. The yield of C₁₋₃ alkanes/olefins was 76.6%.

### Example 2-6 (lower limit of reaction temperature)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 260°C under a reaction pressure of 200 psi. The gas composition analysis of the reaction revealed that, the conversion rate of the carbon dioxide was 26.8%, the yield of carbon monoxide was 3.7%, the yield of methane was 6.7%, the yield of ethene was 2.6%, the yield of ethane was 1.1%, and the yield of propene/propane was 12.8%. The yield of C₁₋₃ alkanes/olefins was 23.2%.

### Example 2-7 (extremely high reaction temperature)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 400°C under a reaction pressure of 200 psi. The gas composition analysis of the reaction revealed that, the conversion rate of the carbon dioxide was 74.2%, the yield of carbon monoxide was 8.0%, the yield of methane was 41.3%, the yield of ethene was 7.4%, the yield of ethane was 5.6%, and the yield of propene/propane was 11.6%. The yield of C₁₋₃ alkanes/olefins was 65.9%.

### Example 2-8 (extremely low reaction temperature)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 240°C under a reaction pressure of 200 psi. The gas composition analysis of the reaction revealed that, the conversion rate of the carbon dioxide was 13.3%, the yield of carbon monoxide was 7.0%, the yield of methane was 4.1%, the yield of ethene was 0.7%, the yield of ethane was 0.4%, and the yield of propene/propane was 1.1%. The yield of C₁₋₃ alkanes/olefins was 6.3%.

### Example 2-9 (upper limit of reaction pressure)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of 400 psi. The gas composition analysis of the reaction revealed that, the conversion rate of the carbon dioxide was 68.0%, the yield of carbon monoxide was 1.0%, the yield of methane was 27.1%, the yield of ethene was 15.7%, the yield of ethane was 3.4%, and the yield of propene/propane was 20.8%. The yield of C₁₋₃ alkanes/olefins was 67.0%.

### Example 2-10 (lower limit of reaction pressure)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of 50 psi. The gas composition analysis of the reaction revealed that, the conversion rate of the carbon dioxide was 50.8%, the yield of carbon monoxide was 5.8%, the yield of methane was 18.7%, the yield of ethene was 10.6%, the yield of ethane was 1.9%, and the yield of propene/propane was 13.9%. The yield of C₁₋₃ alkanes/olefins was 45.1%.

### Example 2-11 (extremely low reaction pressure)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of about 0 psi (pressure shown on pressure meter). The gas composition analysis of the reaction revealed that, the conversion rate of the carbon dioxide was 30.2%, the yield of carbon monoxide was 4.8%, the yield of methane was 16.7%, the yield of ethene was 4.2%, the yield of ethane was 1.5%, and the yield of propene/propane was 3.0%. The yield of C₁₋₃ alkanes/olefins was 25.4%.

### Example 2-12 (upper limit of total space velocity)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 3000 hr⁻¹ (H₂/CO₂=3.5/1, mol/mol) was introduced into the reactor at a reaction temperature of 320°C under a reaction pressure of about 200 psi. The gas composition analysis of the reaction revealed that, the conversion rate of the carbon dioxide was 52.5%, the yield of carbon monoxide was 2.2%, the yield of methane was 22.0%, the yield of ethene was 9.5%, the yield of ethane was 3.2%, and the yield of propene/propane was 15.9%. The yield of C₁₋₃ alkanes/olefins was 50.6%.

### Example 2-13 (lower limit of total space velocity)

The iron-based catalyst in Example 2-1 was sieved, and 9 mL of the iron-based catalyst with 12 mesh to 20 mesh was filled into a reactor of 3/8 inch. A mixture of hydrogen and carbon dioxide having a gas hourly space velocity of 300 hr⁻¹ (H₂/CO₂=3.5/1, mol/mol) was introduced into the reactor at a reaction temperature of 320°C under a reaction pressure of about 200 psi. The gas composition analysis of the reaction revealed that, the conversion rate of the carbon dioxide was 59.5%, the yield of carbon monoxide was 2.1%, the yield of methane was 26.9%, the yield of ethene was 11.3%, the yield of ethane was 3.8%, and the yield of propene/propane was 15.4%. The yield of C₁₋₃ alkanes/olefins was 57.4%.

### Example 2-14 (without potassium carbonate)

The iron-based support of porous FeO(OH)ₓ without potassium carbonate loaded thereon in Example 1 was sieved, and 9 mL of the porous FeO(OH)ₓ powder with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of about 200 psi. The gas composition analysis of the reaction revealed that, the conversion rate of the carbon dioxide was 14.8%, the yield of carbon monoxide was 0.1%, the yield of methane was 14.4%, the yield of ethene was 0.0%, the yield of ethane was 0.2%, and the yield of propene/propane was 0.1%. The yield of C₁₋₃ alkanes/olefins was 14.7%.

### Example 2-15 (excessive potassium carbonate)

The iron-based support of porous FeO(OH)ₓ in Example 1 was added into a potassium carbonate aqueous solution, such that the potassium carbonate was loaded on the porous FeO(OH)ₓ. The above substance was then baked and shaped to obtain an iron-based catalyst, wherein the molar percentage of the FeO(OH)ₓ was 60 mol%, and that of the potassium carbonate was 40 mol%.

The iron-based catalyst was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 320°C under a reaction pressure of about 200 psi. The gas composition analysis of the reaction revealed that, the conversion rate of the carbon dioxide was 16.5%, the yield of carbon monoxide was 7.1%, the yield of methane was 5.3%, the yield of ethene was 1.2%, the yield of ethane was 0.8%, and the yield of propene/propane was 2.2%. The yield of C₁₋₃ alkanes/olefins was 9.5%.

### Comparative Example 1

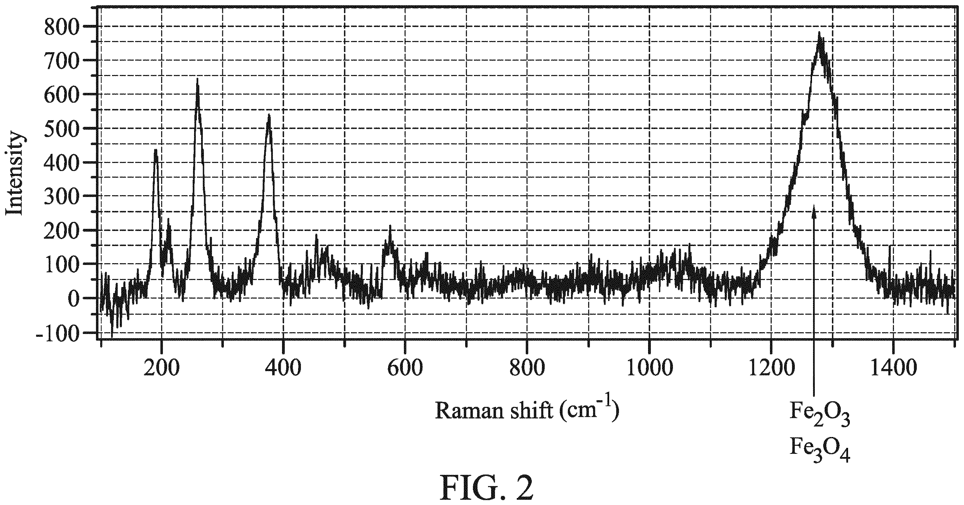

An iron nitrate aqueous solution and ammonia were mixed, and the pH value of the mixture was then adjusted to 9 and rested for 120 minutes and then filtered. The filtered cake was washed out with water and then baked at 110°C to obtain an iron-based support such as porous FeO(OH)ₓ, Fe₂O₃, and Fe₃O₄. The Raman spectrum of the iron-based support is shown in Fig. 2, which includes a large amount of Fe₂O₃ and Fe₃O₄. The obtained iron-based support had a specific surface area of 41.6 m²/g (measured and calculated by BET method, such as nitrogen adsorption at constant temperature), a pore volume of 0.24 cm³/g (measured and calculated by BJH method, such as nitrogen adsorption at constant temperature), and an average pore size of 177 Å (measured and calculated by BJH method, such as nitrogen adsorption at constant temperature). The iron-based support such as FeO(OH)ₓ, Fe₂O₃, and Fe₃O₄ was then added into a potassium carbonate aqueous solution, such that the potassium carbonate was loaded on the iron-based support. The above substance was then baked and shaped to obtain an iron-based catalyst, wherein the molar percentage of the iron-based support such as FeO(OH)ₓ, Fe₂O₃, and Fe₃O₄ was 95 mol%, and that of the potassium carbonate was 5 mol%.

The iron-based catalyst was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 350°C under a reaction pressure of about 200 psi. The gas composition analysis of the reaction revealed that, the conversion rate of the carbon dioxide was 22.2%, the yield of carbon monoxide was 20.9%, the yield of methane was 0.9%, the yield of ethene was 0.3%, the yield of ethane was 0.2%, and the yield of propene/propane was 0%. The yield of C₁₋₃ alkanes/olefins was 1.4%.

### Comparative Example 2

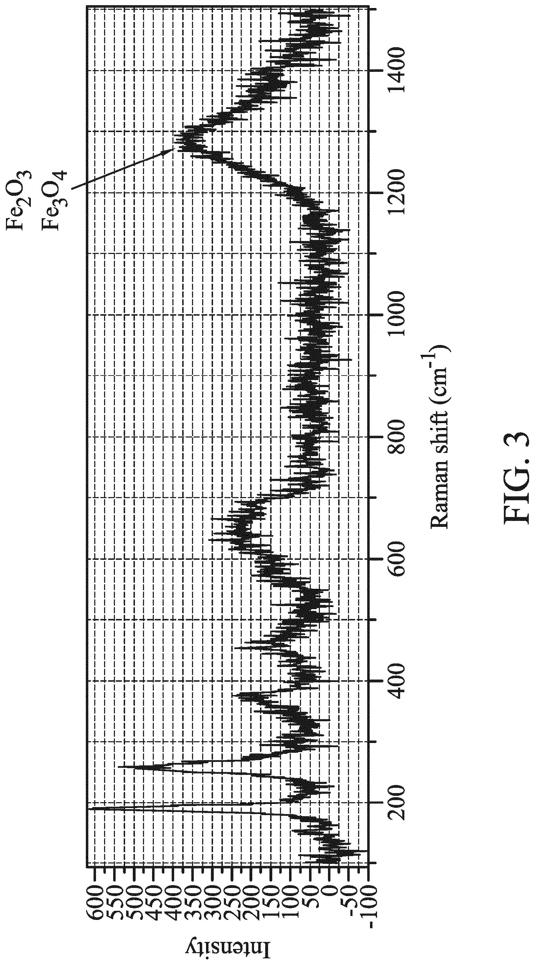

A16267 commercially available from Alfa (iron (III) hydroxide, alpha-phase, 99+% FeO(OH), CAS: 20334-49-4) serving as an iron-based support, and the Raman spectrum of the A16267 is shown in Fig. 3, which includes porous FeO(OH)ₓ, Fe₂O₃, and Fe₃O₄. The iron-based support A16267 commercially available from Alfa had a specific surface area of 14.3 m²/g (measured and calculated by BET method, such as nitrogen adsorption at constant temperature), a pore volume of 0.06 cm³/g (measured and calculated by BJH method, such as nitrogen adsorption at constant temperature), and an average pore size of 166 Å (measured and calculated by BJH method, such as nitrogen adsorption at constant temperature). The iron-based support was then added into a potassium carbonate aqueous solution, such that the potassium carbonate was loaded on the iron-based support. The above substance was then baked and shaped to obtain an iron-based catalyst, wherein the molar percentages of the iron-based support such as FeO(OH)ₓ, Fe₂O₃, and Fe₃O₄ was 95 mol%, and that of the potassium carbonate was 5 mol%.

The iron-based catalyst was sieved, and 9 mL of the iron-based catalyst with 12 mesh to 20 mesh was filled into a reactor of 3/8 inch. A mixture of hydrogen and carbon dioxide having a gas hourly space velocity of 1800 hr⁻¹ (H₂/CO₂=4/1, mol/mol) was introduced into the reactor at a reaction temperature of 400°C under a reaction pressure of about 200 psi. The gas composition analysis of the reaction revealed that, the conversion rate of the carbon dioxide was 25.7%, the yield of carbon monoxide was 24.5%, the yield of methane was 0.6%, the yield of ethene was 0.4%, the yield of ethane was 0 %, and the yield of propene/propane was 0.2%. The yield of C₁₋₃ alkanes/olefins was 1.2%. The above experiment was repeated except the reaction temperature being decreased to 350°C, resulted in the conversion rate of the carbon dioxide <10%.

### Example 3-1

The iron-based catalyst of Example 2-1 (75 mol% of FeO(OH)ₓ, wherein x=1.52, and 25 mol% of potassium carbonate) was sieved, 4.5 mL of the iron-based catalyst with 12 mesh to 20 mesh was filled into a first reactor of 3/8 inch, and 4.5 mL of the iron-based catalyst with 12 mesh to 20 mesh was filled into a second reactor of 3/8 inch. A gas-liquid separation tank was built between the first reactor and the second reactor for separating the gas and the liquid generated from the first reactor and introducing the separated gas into the second reactor. The temperature of the gas-liquid separation tank was set as 50°C.

A mixture of hydrogen and carbon dioxide having a gas hourly space velocity of 1500 hr⁻¹ (H₂/CO₂=8/1, mol/mol) was introduced into the first reactor at a reaction temperature of 320°C under a reaction pressure of 290 psi to generate a gas and a liquid. The gas and the liquid were separated by the gas-liquid separation tank, and the gas was introduced into the second reactor at a reaction temperature of 320°C under a reaction pressure of 290 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 96.0%, the yield of carbon monoxide was 0.5%, the yield of methane was 29.5%, the yield of ethene was 11.5%, the yield of ethane was 5.5%, and the yield of propene/propane was 19.8%. The yield of C₄ alkanes/olefins was 11.9%, the yield of the gaseous alkanes/olefins having a carbon number greater than 4 (C₄₊) was 14.7%, and the yield of the liquid alkanes/olefins having a carbon number ≥ 5 was 2.68%. The total yield of the C₂₋₄ gaseous alkanes/olefins was 48.7%.

### Example 3-2

The first reactor, the second reactor, and the gas-liquid separation tank in Example 3-2 were the same as those in Example 3-1.

A mixture of hydrogen and carbon dioxide having a gas hourly space velocity of 1500 hr⁻¹ (Hz/COz=3.0/1, mol/mol) was introduced into the first reactor at a reaction temperature of 320°C under a reaction pressure of 290 psi to generate a gas and a liquid. The gas and the liquid were separated by the gas-liquid separation tank, and the gas was introduced into the second reactor at a reaction temperature of 320°C under a reaction pressure of 290 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 97.6%, the yield of carbon monoxide was 0.3%, the yield of methane was 30.4%, the yield of ethene was 11.4%, the yield of ethane was 6.4%, and the yield of propene/propane was 21%. The yield of C₄ alkanes/olefins was 12.4%, the yield of the gaseous alkanes/olefins having a carbon number greater than 4 (C₄₊) was 14.1%, and the yield of the liquid alkanes/olefins having a carbon number ≥ 5 was 1.74%. The total yield of the C₂₋₄ gaseous alkanes/olefins was 51.2%.

### Example 3-3

The first reactor, the second reactor, and the gas-liquid separation tank in Example 3-3 were the same as those in Example 3-1.

A mixture of hydrogen and carbon dioxide having a gas hourly space velocity of 1500 hr⁻¹ (H₂/CO₂=3.4/1, mol/mol) was introduced into the first reactor at a reaction temperature of 320°C under a reaction pressure of 290 psi to generate a gas and a liquid. The gas and the liquid were separated by the gas-liquid separation tank, and the gas was introduced into the second reactor at a reaction temperature of 320°C under a reaction pressure of 290 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 98.0%, the yield of carbon monoxide was 0.1%, the yield of methane was 31.2%, the yield of ethene was 11.5%, the yield of ethane was 6.6%, and the yield of propene/propane was 21.4%. The yield of C₄ alkanes/olefins was 12.7%, the yield of the gaseous alkanes/olefins having a carbon number greater than 4 (C₄₊) was 14.5%, and the yield of the liquid alkanes/olefins having a carbon number ≥ 5 was 0%. The total yield of the C₂₋₄ gaseous alkanes/olefins was 52.2%.

### Example 3-4

The first reactor, the second reactor, and the gas-liquid separation tank in Example 3-4 were the same as those in Example 3-1.

A mixture of hydrogen and carbon dioxide having a gas hourly space velocity of 1500 hr⁻¹ (Hz/COz=4.0/1, mol/mol) was introduced into the first reactor at a reaction temperature of 320°C under a reaction pressure of 290 psi to generate a gas and a liquid. The gas and the liquid were separated by the gas-liquid separation tank, and the gas was introduced into the second reactor at a reaction temperature of 320°C under a reaction pressure of 290 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 99.5%, the yield of carbon monoxide was 0%, the yield of methane was 33.1%, the yield of ethene was 11.1%, the yield of ethane was 7.1%, and the yield of propene/propane was 21.1%. The yield of C₄ alkanes/olefins was 12.3%, the yield of the gaseous alkanes/olefins having a carbon number greater than 4 (C₄₊) was 14.9%, and the yield of the liquid alkanes/olefins having a carbon number ≥ 5 was 0%. The total yield of the C₂₋₄ gaseous alkanes/olefins was 51.6%.

### Example 4-1

The iron-based catalyst of Example 1 (95 mol% of FeO(OH)ₓ, wherein x=1.52, and 5 mol% of potassium carbonate) was sieved, 4.5 mL of the iron-based catalyst with 12 mesh to 20 mesh was filled into a first reactor of 3/8 inch, and 4.5 mL of the iron-based catalyst with 12 mesh to 20 mesh was filled into a second reactor of 3/8 inch. A gas-liquid separation tank was built between the first reactor and the second reactor for separating the gas and the liquid generated from the first reactor and introducing the separated gas into the second reactor. The temperature of the gas-liquid separation tank was set as 50°C.

A mixture of hydrogen and carbon dioxide having a gas hourly space velocity of 1500 hr⁻¹ (Hz/COz=3.0/1, mol/mol) was introduced into the first reactor at a reaction temperature of 310°C under a reaction pressure of 200 psi to generate a gas and a liquid. The gas and the liquid were separated by the gas-liquid separation tank, and the gas was introduced into the second reactor at a reaction temperature of 310°C under a reaction pressure of 200 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 83.6%, the yield of carbon monoxide was 0.9%, the yield of methane was 19.8%, the yield of ethene was 6.3%, the yield of ethane was 6.0%, and the yield of propene/propane was 15.8%. The yield of C₄ alkanes/olefins was 9.1%, the yield of the gaseous alkanes/olefins having a carbon number greater than 4 (C₄₊) was 6.2%, and the yield of the liquid alkanes/olefins having a carbon number ≥ 5 was 19.5%. The total yield of the C₂₋₄ gaseous alkanes/olefins was 37.2%.

### Example 4-2

The first reactor, the second reactor, and the gas-liquid separation tank in Example 4-2 were the same as those in Example 4-1.

A mixture of hydrogen and carbon dioxide having a gas hourly space velocity of 1500 hr⁻¹ (H₂/CO₂=3.5/1, mol/mol) was introduced into the first reactor at a reaction temperature of 310°C under a reaction pressure of 200 psi to generate a gas and a liquid. The gas and the liquid were separated by the gas-liquid separation tank, and the gas was introduced into the second reactor at a reaction temperature of 310°C under a reaction pressure of 200 psi to generate a gaseous product and a liquid product. As known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 94.6%, the yield of carbon monoxide was 1.3%, the yield of methane was 20.8%, the yield of ethene was 8.9%, the yield of ethane was 4.7%, and the yield of propene/propane was 17.6%. The yield of C₄ alkanes/olefins was 10.7%, the yield of the gaseous alkanes/olefins having a carbon number greater than 4 (C₄₊) was 7.8%, and the yield of the liquid alkanes/olefins having a carbon number ≥ 5 was 22.8%. The total yield of the C₂₋₄ gaseous alkanes/olefins was 41.9%.

### Example 4-3

The first reactor, the second reactor, and the gas-liquid separation tank in Example 4-3 were the same as those in Example 4-1.

A mixture of hydrogen and carbon dioxide having a gas hourly space velocity of 1500 hr⁻¹ (H₂/CO₂=3.5/1, mol/mol) was introduced into the first reactor at a reaction temperature of 310°C under a reaction pressure of 200 psi to generate a gas and a liquid. The gas and the liquid were separated by the gas-liquid separation tank, and the gas was introduced into the second reactor at a reaction temperature of 310°C under a reaction pressure of 200 psi to generate a gaseous product and a liquid product. After continuously reacting for 2008 hours, as known from the composition analysis of the liquid (after being dried to remove water thereof) separated from the gas-liquid separation tank, and the composition analysis of the gaseous product and the liquid product (after being dried to remove water thereof) generated from the second reactor, the conversion rate of the carbon dioxide was 94.9%, the yield of carbon monoxide was 1.2%, the yield of methane was 21.7%, the yield of ethene was 9.0%, the yield of ethane was 4.7%, and the yield of propene/propane was 17.6%. The yield of C₄ alkanes/olefins was 10.5%, the yield of the gaseous alkanes/olefins having a carbon number greater than 4 (C₄₊) was 7.4%, and the yield of the liquid alkanes/olefins having a carbon number ≥ 5 was 22.8%. The total yield of the C₂₋₄ gaseous alkanes/olefins was 41.8%.

It will be apparent to those skilled in the art that various modifications and variations can be made to the disclosed methods and materials. It is intended that the specification and examples be considered as exemplary only, with the true scope of the disclosure being indicated by the following claims and their equivalents.

## Claims

1. An iron-based catalyst, comprising:
70 mol% to 97 mol% of porous FeO(OH)ₓ, wherein 1<x<2; and
3 mol% to 30 mol% of alkaline metal compound loaded on the porous FeO(OH)ₓ.

2. The iron-based catalyst as claimed in claim 1, wherein the porous FeO(OH)ₓ has a specific surface area of at least 100 m²/g.

3. The iron-based catalyst as claimed in claim 1, wherein the porous FeO(OH)ₓ has a pore volume of 0.2 cm³/g to 0.5 cm³/g.

4. The iron-based catalyst as claimed in claim 1, wherein the porous FeO(OH)ₓ has an average pore size of 40 Å to 70 Å.

5. The iron-based catalyst as claimed in claim 1, wherein the alkaline metal compound comprises sodium carbonate, potassium carbonate, sodium hydroxide, potassium hydroxide, sodium oxide, potassium oxide, or a combination thereof.

6. A method of hydrogenating carbon dioxide, comprising:
(i) contacting carbon dioxide and hydrogen with an iron-based catalyst to form a liquid and a gas,
wherein the liquid comprises CₙH₂ₙ, CₙH₂ₙ₊₂, or a combination thereof and water, and n is 5 to 18,
wherein the gas comprises CH₄, CₘH₂ₘ, CₘH₂ₘ₊₂, or a combination thereof, hydrogen, and carbon dioxide, and m is 2 to 9,
wherein the iron-based catalyst comprises:
70 mol% to 97 mol% of porous FeO(OH)ₓ, wherein 1<x<2; and
3 mol% to 30 mol% of alkaline metal compound loaded on the porous FeO(OH)ₓ.

7. The method as claimed in claim 6, wherein the hydrogen and the carbon dioxide contacting the iron-based catalyst have a ratio of 2:1 to 4:1, and the hydrogen and the carbon dioxide contacting the iron-based catalyst have a gas hourly space velocity of 300 hr⁻¹ to 3000 hr⁻¹.

8. The method as claimed in claim 6, wherein the step of contacting the carbon dioxide and the hydrogen with the iron-based catalyst is performed under a pressure of 50 psi to 400 psi at a temperature of 260°C to 360°C.

9. The method as claimed in claim 6, further comprising:
(ii) separating the gas and the liquid; and
(iii) contacting the gas with another iron-based catalyst to form another liquid and another gas,
wherein the other liquid comprises CₙH₂ₙ, CₙH₂ₙ₊₂, or a combination thereof and water, and n is 5 to 18,
wherein the other gas comprises CH₄, CₘH₂ₘ, CₘH₂ₘ₊₂, or a combination thereof, hydrogen, and carbon dioxide, and m is 2 to 9,
wherein the other iron-based catalyst comprises:
70 mol% to 97 mol% of another porous FeO(OH)ₓ, wherein 1<x<2; and
3 mol% to 30 mol% of another alkaline metal compound loaded on the other porous FeO(OH)ₓ.

10. The method as claimed in claim 9, wherein the conversion rate of carbon dioxide in step (iii) is higher than the conversion rate of carbon dioxide in step (i).

11. The method as claimed in claim 9, further comprising repeating step (ii) and step (iii) several times.
